Europäisches Patentamt

European Patent Office

Office européen des brevets

(19)

(11) Veröffentlichungsnummer: **0 179 423**
**B1**

(12) **EUROPÄISCHE PATENTSCHRIFT**

(45) Veröffentlichungstag der Patentschrift: **11.04.90**

(21) Anmeldenummer: **85113317.3**

(22) Anmeldetag: **21.10.85**

(51) Int. Cl.⁵: **A 63 B 21/06,** A 61 N 5/06

(54) Liege-Einrichtung für Bewegungstherapien.

(30) Priorität: **26.10.84 CH 5135/84**

(43) Veröffentlichungstag der Anmeldung:
**30.04.86 Patentblatt 86/18**

(45) Bekanntmachung des Hinweises auf die
Patenterteilung:
**11.04.90 Patentblatt 90/15**

(84) Benannte Vertragsstaaten:
**AT BE DE FR GB IT NL SE**

(56) Entgegenhaltungen:
**DE-A-2 414 722**
**DE-A-3 033 419**
**GB-A-2 128 488**

(73) Patentinhaber: **Ruf, Inge**
**Toggwilerstrasse 81**
**CH-8760 Meilen (CH)**

(72) Erfinder: **Ruf, Inge**
**Toggwilerstrasse 81**
**CH-8760 Meilen (CH)**

(74) Vertreter: **Petschner, Goetz**
**Patentanwaltsbüro G. Petschner Seidengasse 18**
**CH-8001 Zürich (CH)**

Courier Press, Leamington Spa, England.

## Beschreibung

Die vorliegende Erfindung betrifft eine Liege-Einrichtung für Bewegungstherapien.

Im Rahmen der Gesundheitspflege sind Liege-Einrichtungen bekannt geworden, die dem Benützer gestatten, in liegender Haltung gymnastische Uebungen unter vorgegebener erhöhter Umgebungstemperatur auszuführen.

Bekannt ist in diesem Zusammenhang gemäss UK-Patentanmeldung 2,128,488 eine Anordnung, bei welcher die Liege an ihrer Oberseite mit Luftaustrittsöffnungen versehen ist, die an ein Gebläse angeschlossen sind, wobei unter dem Ueberdruck der Gebläseluft eine flexible Hülle über der Liege aufgeblasen wird. Diese Hülle bildet dann einen, den Körper des Benutzers aufnehmenden, dessen Kopf freilassenden Hohlraum oberhalb der Liege.

Bei einer anderen Anordnung ist die aufblasbare Hülle durch eine starre, kuppelartige Haube ersetzt.

Beide Ausführungsformen sind aber nur bedingt brauchbar. So ist der Benutzer einem ständigen Gebläseluftstrom ausgesetzt, was sicher nicht immer als angenehm empfunden wird. Zudem beschränkt die Hülle oder Haube entschieden die Bewegungsfreiheit des Benützers, was eine Anwendung bei Menschen, die unter Klaustro-Phobie leiden, völlig ausschliesst.

Aufgabe der vorliegenden Erfindung ist es nun, eine Liege-Einrichtung der vorgenannten Art zu schaffen, die dem Benutzer optimale Bewegungsfreiheit gewährt unter Aufrechterhaltung einer vorgegebenen erhöhten Umgebungstemperatur.

Dies lässt sich erfindungsgemäss erreichen durch eine, von einer ausserhalb des Bewegungsbereiches installierten IR-Lampenanordnung erzeugte Wärmestrahlenglocke über dem Auflagebereich der Liege.

Durch diese Massnahmen wird der Benutzer nun nicht mehr durch eine ihn umgebende Hülle oder Haube eingeengt und nicht mehr einem Gebläseluftstrom ausgesetzt. Der Benutzer liegt nunmehr unter einer durch Licht erzeugten Wärmeglocke, hat volle Bewegungsfreiheit und kann zudem den Bereich der Liege jederzeit verlassen. Zudem wird der Benutzer bei seinen Uebungen vom wirksamen, gesundheitsfördernden Infrarot-Licht bestrahlt oder er kann gleichzeitig unter den hautfreundlichen und wärmenden Ultraviolett-A-Strahlen ungefährdet bräunen.

Eine solche erfindungsgemässe Liege-Einrichtung kann in jedem Raum untergebracht werden, wofür es zweckmässig ist, wenn sich die Lampenanordnung an einem Stativ oder an einer Zimmerwand oder -decke befestigen lässt.

Durch geeignete Parabolic-Reflektoren ist die flächige Ausdehnung der Wärmestrahlenglocke bestimmt.

Für die Durchführung von Gymnastikübungen ist es vorteilhaft, wenn in ansich bekannter Weise die Liege mit Schlaufen und/oder Handgriffen ausgerüstet ist, welche gegen einen Widerstand in Form von Federn und/oder Gewichten bewegbar sind.

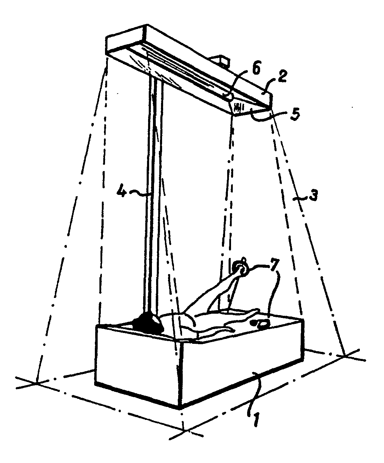

Eine beispielsweise Ausführungsform des Erfindungsgegenstandes ist nachfolgend anhand der Zeichnung, welche in schaubildartiger Darstellung eine erfindungsgemässe Liege-Einrichtung für Bewegungstherapien zeigt, näher erläutert.

Die erfindungsgemässe Liege-Einrichtung besteht zunächst aus einer ansich bekannten Liege 1 mis beispielsweise Schlaufen 7 im Fussbereich und in der Regel mit hier näher gezeigten Handgriffen im Handbereich der Liegefläche, welche vom Benutzer gegen einen Widerstand in Form von Federn und/oder Gewichten im Innern der Liege bewegbar sind.

Erfindungsgemäss wird nun der Benutzer von einer ausreichend grossen Wärmestrahlenglocke 3 umhüllt, welche von einer IR- und/oder UVA-Lampenanordnung 2 erzeugt wird, wobei geeignete Parabolic-Reflektoren 5 die flächige Ausdehnung der Wärmestrahlenglocke 3 bestimmen, wie das durch die gestrichelten bzw. strichpunktierten Strahlengänge angedeutet ist.

Eine solche Lampenanordnung kann normale Infrarot-Lampen 6 oder UVA-Lampen umfassen, wie etwa die HPA-400-W-Lampen von Philips (Marke).

Eine grosse Mobilität der erfindungsgemässen Liege-Einrichtung wird erreicht, wenn die Lampenanordnung 2 an einem Stativ installiert wird. Natürlich kann die Lampenanordnung 2 aber auch an einer Zimmerwand oder -decke befestigt werden.

Aus dem Vorbeschriebenen ergibt sich somit eine Liege-Einrichtung, welche vergleichsweise den bekannten Anordnungen eine wesentlich vielseitigere, wirksamere und bedeutend angenehmere Benutzung zulässt.

## Patentansprüche

1. Liege-Einrichtung für Bewegungstherapien, gekennzeichnet durch die Kombination einer Liege (1), welche mit Zugmitteln (7) ausgerüstet ist, die gegen den Widerstand von Federmitteln bewegbar sind, sowie einer IR-Lampenanordnung (2) über dem Auflagebereich der Liege ausserhalb des therapeutischen Bewegungsbereiches zur Erzeugung einer Wärmestrahlenglocke (3) über der Liegefläche, wobei die flächige Ausdehnung der Wärmestrahlenglocke im Bereich der Liegefläche durch Parabolic-Reflektoren (5) an der Lampenanordnung (2) bestimmt ist.

2. Liege-Einrichtung nach Anspruch 1, dadurch gekennzeichnet, dass die Lampenanordnung (2) an einem Stativ (4), oder an einer Zimmerwand oder -decke befestigt ist.

## Revendications

1. Table pour exercices thérapeutiques, caractérisée par la combinaison d'une table (1) comprenent des moyens de traction (7) pouvant être déplacés à l'encontre de la résistance de moyens tels que des ressorts, ainsi qu'une disposition de lampes infrarouges (2) prévues au-dessus de la surface de repos de la table, extérieure à la zone de mouvement thérapeutique, pour produire une cloche (3) de radiation thermique au-dessus de la surface de repos, l'étendue plane des radiations thermique de la cloche étant déterminée dans la zone de la surface de repos par des réflecteurs paraboliques (5) prévus dans la disposition des lampes (2).

2. Table selon la revendication 1, caractérisée en ce que l'ensemble des lampes (2) est fixé sur un support (4), ou sur une paroi de la pièce ou sur le plafond de cette dernière.

**Claims**

1. Bench arrangement for kinetotherapy, characterized by the combination of a bench (1) equipped with drawing or pulling means (7) which are moveable against the resistance of spring means, as well as an IR-lamp arrangement (2) above the supporting area of the bench and outside the therapeutic area of movement for producing a thermal radiation cover (3) over the surface of the bench; the plane of extent of the thermal radiation cover in the region of the supporting area of the bench being defined by parabolic reflectors (5) on the lamp arrangement (2).

2. The bench arrangement as defined in claim 1, characterized in that the lamp arrangement (2) is mounted on a support (4) or a wall or ceiling of a room.